# EUROPEAN PATENT APPLICATION

(11) **EP 3 093 959 A1**
(43) Date of publication of application: **16.11.2016**
(21) Application number: 16169311.4
(22) Date of filing: 12.05.2016
(51) Int. Cl.: H02J 50/90, H02J 50/12

(54) **WIRELESS POWER RECEIVER AND METHOD FOR USE IN A WIRELESS POWER RECEIVER**

(30) Priority: 12.05.2015 US 201562159985 P
(71) Applicant: Powermat Technologies Ltd., 90850 Neve Ilan (IL)
(72) Inventor: GLUZMAN, Ilya, 58670 Holon (IL); MACH, Elieser, 90938 Rosh Tzurim (IL); MOSHKOVICH, Oz, 76452 Rehovot (IL); GREENWALD, Oola, 90805 Mevasseret Zion (IL); RAVEH, Guy, 99870 Mataa (IL); KDOSHIM, Moti, 7176090 Modiin (IL); SALHUV, Amir, 7648841 Rehovot (IL)
(74) Representative: Beck & Rössig European Patent Attorneys

(57) **Abstract**

The disclosure herein relates relate to systems and methods for wireless power transfer between a wireless power outlet and a wireless power receiver. In particular the disclosure relate to a wireless power receiver enabled to monitor a sensor arrangement associated with the secondary coil of the power receiver and further determine its orientation relative to the wireless power outlet and calculate the electric power loss for an orientation. Furthermore, a monitoring unit associated with the wireless power receiver is operable to receive power transfer and orientation data from the sensor arrangement such as voltage, current, capacity, resistance, temperature and the like, to calculate efficiency data from the sensor data and further operable to communicate the efficiency data to a communication and control.

## Description

### FIELD OF THE INVENTION

The invention relates to a wireless power receiver and a method for use in a wireless power receiver. More specifically, the invention relates to transfer of power between a wireless power outlet and a wireless power receiver. In particular the invention relates to a wireless power receiver enabled to monitor a sensor arrangement associated with the secondary coil of the power receiver and further determine its orientation relative to the wireless power outlet and calculate the electric power loss for an orientation.

### BACKGROUND OF THE INVENTION

The spread of mobile devices such as mobile handsets, media players, tablet computers and laptops/notebooks/netbooks and ultra-books increases user demand for access to power points at which they may transfer power to charge mobile electrical devices while out and about or on the move. Thus, systems that conveniently provide the opportunity to transfer power for charging the electrical devices in public spaces, in which the user of a mobile electrical device may remain for extended periods of time, say more than a few minutes or so, such as restaurants, coffee shops, airport lounges, trains, buses, taxis, sports stadia, auditoria, theatres, cinemas or the like, becomes a basic necessity.

Inductive power coupling allows energy to be transferred from a power supply to an electric load without a wired connection therebetween. An oscillating electric potential is applied across a primary inductor. This sets up an oscillating magnetic field in the vicinity of the primary inductor. The oscillating magnetic field may induce a secondary oscillating electrical potential in a secondary inductor placed close to the primary inductor. In this way, electrical energy may be transmitted from the primary inductor to the secondary inductor by electromagnetic induction without a conductive connection between the inductors.

When electrical energy is transferred from a primary inductor to a secondary inductor, the inductors are said to be inductively coupled. An electric load wired in series with such a secondary inductor may draw energy from the power source wired to the primary inductor when the secondary inductor is inductively coupled thereto.

Furthermore, in wireless power systems, displacement of the power receiver (Rx) may result in change of performance due to the change in coupling and in electrical losses on metallic components comprising the wireless power transmitter (Tx) and the power receiver (Rx) sides, thus it is required that the power receiver coil position relative to the power transmitter coil is determined. Thus, with the growing variability of power receiver devices, embedded into different electrical hosting devices (smart phones, tablets, laptops and the like) a new challenge is being presented, in particular, as the performance is influenced by the various parameters associated with the orientation (location, direction and displacement) of the secondary coil relative to the primary coil. Specifically, applications associated with extended range capabilities, higher power applications and the need in effective foreign object detection (FOD) mechanisms emphasize even further the need in such determination.

It will be appreciated that there is therefore a need for a wireless power transfer system and method enabling a wireless power receiver to determine its orientation and further determine the associated electrical power loss within the wireless power receiver during power transfer.

The present disclosure addresses this need.

### SUMMARY OF THE INVENTION

According to one aspect of the disclosure a wireless power receiver is introduced, connectable with an electrical hosting device and configured to draw power from a wireless power outlet, the wireless power receiver comprising: a secondary coil connectable to an electric load operable to couple with a primary coil associated with said wireless power outlet; a communication and control unit operable to communicate power control signals to the wireless power outlet; a plurality of electrical sensors associated with the secondary coil configured to sense an orientation of the secondary coil relative to the primary coil; and a monitoring unit operable to communicate with a processing unit, the monitoring unit configured to monitor power transfer parameters associated with each of said plurality of electrical sensors; wherein the monitoring unit is operable to receive orientation data from the plurality of electrical sensors, to calculate efficiency data from said sensor data and further operable to communicate the efficiency data to the communication and control unit.

Variously, the plurality of electrical sensors are selected from a group consisting of: a sensing coil, a magnetic field sensor, a temperature sensor, a position sensor and combinations thereof. Accordingly, the orientation data comprises data pertaining to at least one of: at least one parameter determining a location, at least one parameter determining a direction and at least one parameter determining a displacement.

Where appropriate, the efficiency data determines an estimated level of electrical power loss at the current orientation of the wireless power receiver.

According to some embodiments, the wireless power receiver is further operable to determine a desired orientation such that the estimated electrical power loss is less than a pre-configured efficiency threshold. Additionally, the communication and control unit of the wireless power receiver is further configured to provide a user indication of the orientation. Furthermore, the wireless power receiver is operable to determine a desired orientation such that the estimated electrical power loss is beneath a pre-configured efficiency threshold.

Variously, the power transfer parameters are selected from a group consisting of: a voltage parameter, a current parameter, a capacitance parameter, a resistance parameter, a temperature value and combinations thereof.

Optionally, the processing unit associated with the wireless power receiver is associated with the electrical hosting device. Alternatively, the processing unit is associated with the wireless power receiver itself.

Where appropriate, the power receiver further comprising a metal shielding between the wireless power receiver and the electrical hosting device; the metal shielding is operable to reduce the electric power loss associated with metallic components of the electrical hosting device beneath an efficiency threshold setting.

According to another aspect of the disclosure, a method is taught for use in a wireless power receiver associated with a hosting electrical device, the wireless power receiver operable to couple with a wireless power transmitter and configured to transmit communication signals to trigger wireless power transfer, the wireless power receiver comprises: a secondary coil connectable to an electric load operable to couple with a primary coil associated with the wireless power outlet; a communication and control unit operable to communicate power control signals to the wireless power outlet; a plurality of electrical sensors associated with the secondary coil; and a plurality of electrical sensors associated with the secondary coil configured to sense an orientation of the secondary coil relative to the primary coil; and a monitoring unit operable to communicate with a processing unit, the monitoring unit configured to monitor power transfer parameters associated with each of the plurality of electrical sensors; the method for operating the wireless power receiver in an improved manner such that the electrical power loss associated with said wireless power receiver during wireless power transfer are beneath a configurable threshold, the method comprising the steps of:
The communication and control unit, coupling with the wireless power transmitter; the communication unit and control, triggering wireless power transfer from the wireless power transmitter; the monitoring unit, receiving orientation data from the plurality of electrical sensors; the monitoring unit, analyzing the power transfer parameters of each of the plurality of electrical sensors; and the monitoring unit, calculating efficiency data from the orientation data received.

Variously, the orientation data comprises data pertaining to at least one of: a location of the secondary coil relative to primary coil; a direction of the secondary coil relative to primary coil; and a displacement of the secondary coil relative to primary coil.

As appropriate, the method further comprising the step of providing an indication of an estimated level of electrical power loss associated with the wireless power receiver, from the calculated efficiency data at the time of wireless power transfer, using a user interface.

As appropriate, the method further comprising the step of providing an indication of a current orientation of the secondary coil associated with the wireless power receiver relative to the primary coil associated with the wireless power transmitter, using a user interface.

As appropriate, the method further comprising the step of calculating an optimized orientation of the secondary coil relative to the primary coil, the optimized orientation having an estimated level of electrical power loss beneath a beneath an efficiency threshold setting.

As appropriate, the method further comprising the step of providing an indication of the optimized orientation, using a user interface.

As appropriate, the method further comprising the step of providing an indication of the estimated level of electrical power loss and said efficiency threshold setting, using a user interface.

Where appropriate, the user interface is configured to use a visual interface or an audio interface.

Variously, the power transfer parameters are selected from a group consisting of: a voltage parameter, a current parameter, a capacitance parameter, a resistance parameter, a temperature parameter and combinations thereof.

### BRIEF DESCRIPTION OF THE DRAWINGS

For a better understanding of the embodiments and to show how it may be carried into effect, reference will now be made, purely by way of example, to the accompanying drawings.

With specific reference now to the drawings in detail, it is stressed that the particulars shown are by way of example and for purposes of illustrative discussion of selected embodiments only, and are presented in the cause of providing what is believed to be the most useful and readily understood description of the principles and conceptual aspects. In this regard, no attempt is made to show structural details in more detail than is necessary for a fundamental understanding; the description taken with the drawings making apparent to those skilled in the art how the several selected embodiments may be put into practice. In the accompanying drawings:
Fig. 1 is a block diagram showing the main elements of an inductive power transfer system with a feedback signal path according to embodiments of the present invention;
Fig. 2 is a graph showing how the amplitude of operational voltage of an inductive power transfer system varies with transmission frequency;
Fig. 3A is a schematic representation of a pin-less power coupling consisting of a pin-less power jack and a pin-less power plug according to another embodiment of the present invention;
Fig. 3B is a schematic representation of an illustrative example of an inductively enabled platform providing power to electrical devices;
Fig. 4A is a block diagram representing the main features of a wireless power outlet locator, according to the presently disclosed subject matter;
Fig. 4B is a schematic representation of a possible wireless power outlet locator with four sensors, according to the presently disclosed subject matter;
Figs. 5 schematically represents partial diagram of an inductive power transmission system showing an inductive power receiver configuration with a sensor arrangement associated with the secondary coil and a monitoring unit of the currently disclosed subject matter;
Fig. 6A is a block diagram showing the main elements of an inductive power receiver comprising a secondary coil coupled with a sensor arrangement of the currently disclosed subject matter;
Figs. 7A-B is a schematic representation of two different orientations of a secondary coil associated with a wireless power receiver relative to a primary coil associated with a wireless power outlet, including the associated sensor arrangement;
Fig. 8 is a schematic diagram representing various possible efficiency related sensor-based analysis; and
Figs. 9A-B are flowcharts representing methods for use in a wireless power receiver wherein the secondary coil is associated with a sensor arrangement and further configured to determine secondary coil orientation.

### DETAILED DESCRIPTION

Aspects of the present invention relate to providing system and methods for a novel mechanism to enable the wireless power receiver to determine the orientation of its secondary coil relative to the primary coil of the wireless power outlet during wireless power transfer, once a coupling is being formed. It is particularly noted that existing methods rely on displacement determination, for a single coil or location determination, for a coil array performed by the wireless power outlet only.

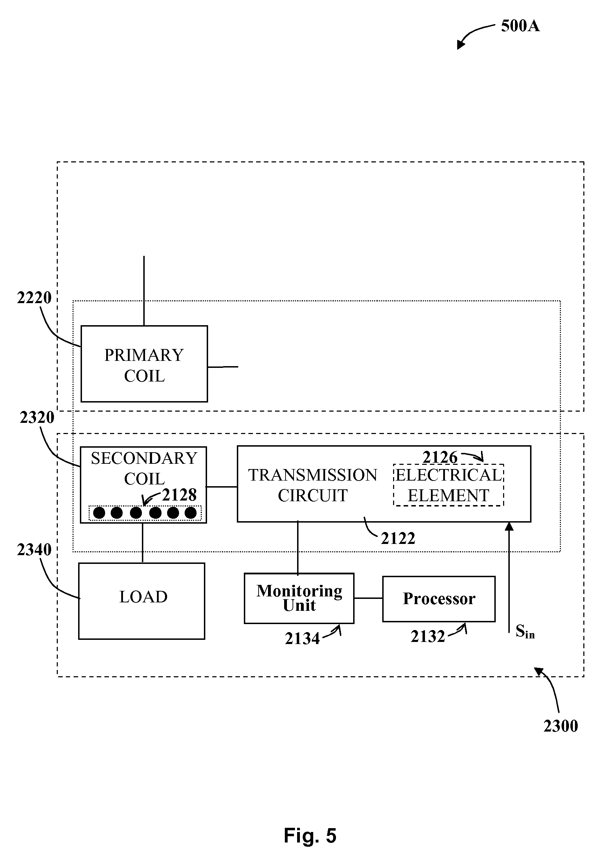

By using a various sensor arrangement in the wireless power receiver, mainly associated with its secondary coil, the orientation (as determined hereinafter) of the secondary coil of the wireless power receiver relative to the wireless power transmitter coil may be determined. Furthermore, by monitoring the power transfer parameters associated with each sensor such as voltage, current, capacity, resistance, temperature and the like and by knowing the location of each sensor, the wireless power receiver may be operable to determine its own orientation. Specifically, the monitoring unit (Fig. 5) is operable to receive orientation data from the sensor arrangement, to calculate efficiency data from the sensor data and further operable to communicate the efficiency data to a communication and control unit of the power receiver.

Additionally, existing methods require the power receiver to estimate the electrical losses on the metallic components both in the power receiver and in the hosting device. As the metallic component's arrangement in the hosting device is not always symmetric, different orientations may result in different levels of electrical losses during power transfer. By using the currently disclosed invention a power receiver is capable to determine its orientation (and associated location, direction and displacement) and further operable to provide accurate estimation for the expected electrical losses for every orientation of the electrical hosting device.

As used herein, the term "orientation" with respect to the current disclosure refers to a combination of a location, a direction and a displacement of the secondary coil associated with a wireless power receiver relative to the primary coil associated with the wireless power transmitter forming a coupling mate and enabling power transfer.

As used herein, the mobile electrical device may be referred to herein as, variously, a 'user device", an "electrical device", an "electronic device", a 'mobile device", a 'communication device" or a 'device". The device may be an electrical device with a battery, e.g., a mobile handset, a media player, a tablet computer, a laptop/notebook/netbook/ultra-book, a PDA or the like. Alternatively, the device may be an accessory with a battery, such as earphones, watches, wearable devices and the like, or a stand-alone battery. As a further alternatively, the device may be any powered device, including electrical devices without a battery.

The portable wireless power transfer unit point may be referred to as, variously, a "wireless power transmitter", a "wireless power outlet", a "PAP", a "hotspot", a "charger" or a "charging spot".

As used herein, the term "memory" or "memory unit" may represent one or more devices for storing data, including read-only memory (ROM), random access memory (RAM), magnetic RAM, core memory, magnetic disk storage mediums, optical storage mediums, flash memory devices or other computer-readable mediums for storing information. The term "computer-readable medium" includes, but is not limited to, portable or fixed storage devices, optical storage devices, wireless channels, a "SIM" card, other smart cards, and various other mediums capable of storing, containing or carrying instructions or data.

It is noted that, where appropriate, the power receiver may use various mechanisms to reduce the electrical power loss in the power receiver, such as a metal shielding between the wireless power receiver and the electrical hosting device, to reduce the electric power loss associated with metallic components of the electrical hosting device beneath an efficiency threshold setting.

Additionally, the design of electrical the hosting device may consider placing the device metallic components as far as possible from the power receiver coil.

### Description of the Embodiments

It is noted that the systems and methods of the invention described herein may not be limited in its application to the details of construction and the arrangement of the components or methods set forth in the description or illustrated in the drawings and examples. The systems, methods of the invention may be capable of other embodiments or of being practiced or carried out in various ways; however, it is to be understood that the disclosed embodiments are merely exemplary of the invention that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present invention.

Alternative methods and materials similar or equivalent to those described herein may be used in practice or testing of embodiments of the invention. Nevertheless, particular methods and materials are described herein for illustrative purposes only. The materials, methods, and examples are not intended to be necessarily limiting.

Accordingly, various embodiments may omit, substitute, or add various procedures or components as appropriate. For instance, it should be appreciated that the methods may be performed in an order different than described, and that various steps may be added, omitted or combined. Also, aspects and components described with respect to certain embodiments may be combined in various other embodiments. It should also be appreciated that the systems, methods, devices, and software may individually or collectively be components of a larger system, wherein other procedures may take precedence over or otherwise modify their application.

The portable wireless power transfer unit may be operable to form an inductive power coupling. The inductive power coupling consists of a primary inductive coil associated with an inductive power outlet of the portable wireless unit and a secondary inductive coil associated with an electrical mobile device, such as a hand held device, a smartphone, a tablet and the like. The primary coil is wired to a power supply, typically via a driver which provides the electronics necessary to drive the primary coil. An oscillating electric potential is applied across the primary coil which induces an oscillating magnetic field therearound.

When the secondary coil is brought within range of the primary coil, the oscillating magnetic field may induce an oscillating electrical current in the secondary coil and the pair of coils may form the inductive coupling such that power is transferred from the primary coil to the secondary coil. According to requirements, the electric potential provided to the primary coil may oscillate at a frequency which is resonant with the secondary coil or may alternatively oscillate at a non-resonant frequency shifted from the natural resonant frequency of the inductive couple formed by the primary coil and the secondary coil.

In this way a portable wireless power transfer unit may provide power to a wireless power receiving device, while in communication with a remote management server. An electric load wired in series with such a secondary coil may draw energy from the power source when the secondary coil is inductively coupled to the primary coil.

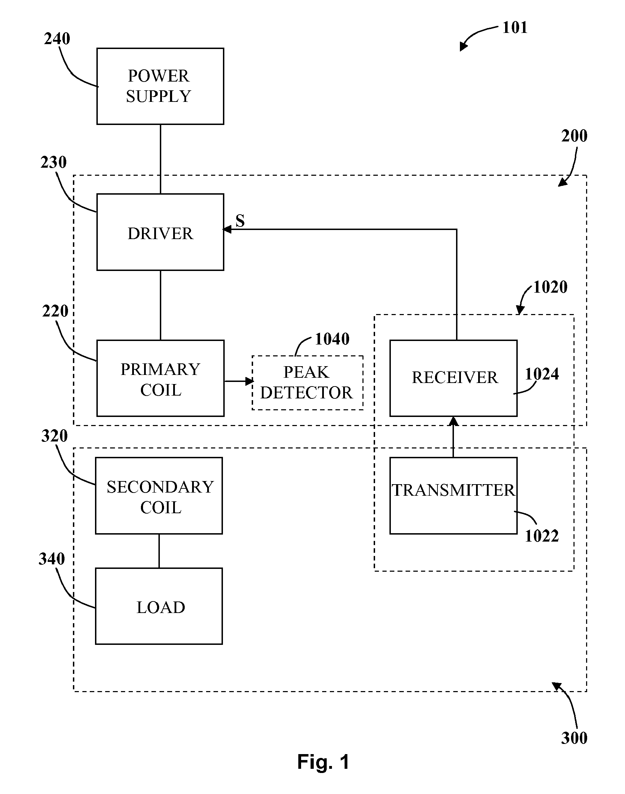

Reference is now made to Fig. 1, there is provided a schematic block diagram of the main elements of an inductive power transfer system, which is generally indicated at 100, for transmitting power at a non-resonant frequency. The inductive power transfer system 100 consists of an inductive power outlet 200 configured to provide power to a remote secondary unit 300. The inductive power outlet 200 includes a primary inductive coil 220 wired to a power source 240 via a driver 230. The driver 230 is configured to provide an oscillating driving voltage to the primary inductive coil 220.

The secondary unit 300 includes a secondary inductive coil 320, wired to an electric load 340, which is inductively coupled to the primary inductive coil 220. The electric load 340 draws power from the power source 240. A communication channel 1020 may be provided between a transmitter 1022 associated with the secondary unit 300 and a receiver 1024 associated with the inductive power outlet 200. The communication channel 120 may provide feedback signals S and the like to the driver 230.

In some embodiments, a voltage peak detector 1040 is provided to detect large increases in the transmission voltage. The peak detector 1040 may be used to detect irregularities such as the removal of the secondary unit 200, the introduction of power drains, short circuits or the like.

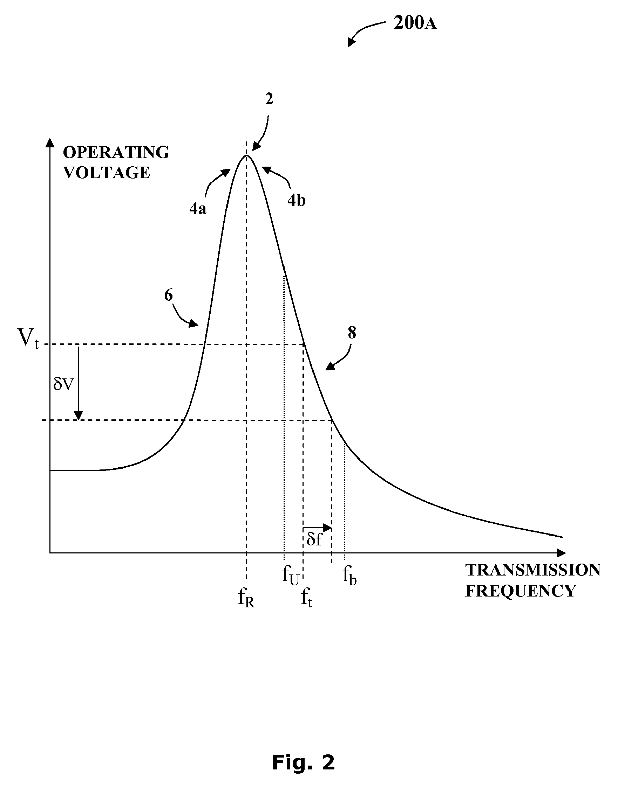

Fig. 2 is a graph showing how the amplitude of the operational voltage of an inductive power transfer system varies according to the transmission frequency. It is noted that the voltage is at its highest when the transmission frequency is equal to the resonant frequency f_{R} of the system, this maximum amplitude is known as the resonance peak 2. It is further noted that the slope of the graph is steepest in the regions 4a, 4b to either side of the resonance peak 2. Thus in inductive transfer systems, which operate at or around resonance, a small variation in frequency results in a large change in induced voltage. Similarly, a small change in the resonant frequency of the system results in a large change in the induced voltage. For this reason prior art resonant inductive transfer systems are typically very sensitive to small fluctuations in environmental conditions or variations in alignment between the induction coils.

It is a particular feature of embodiments of the current invention that the driver 230 (Fig. 1) is configured and operable to transmit a driving voltage which oscillates at a transmission frequency which is substantially different from the resonant frequency of the inductive couple. Optionally the transmission frequency is selected to lie within one of the near-linear regions 6, 8 where the slope of the frequency-amplitude graph is less steep.

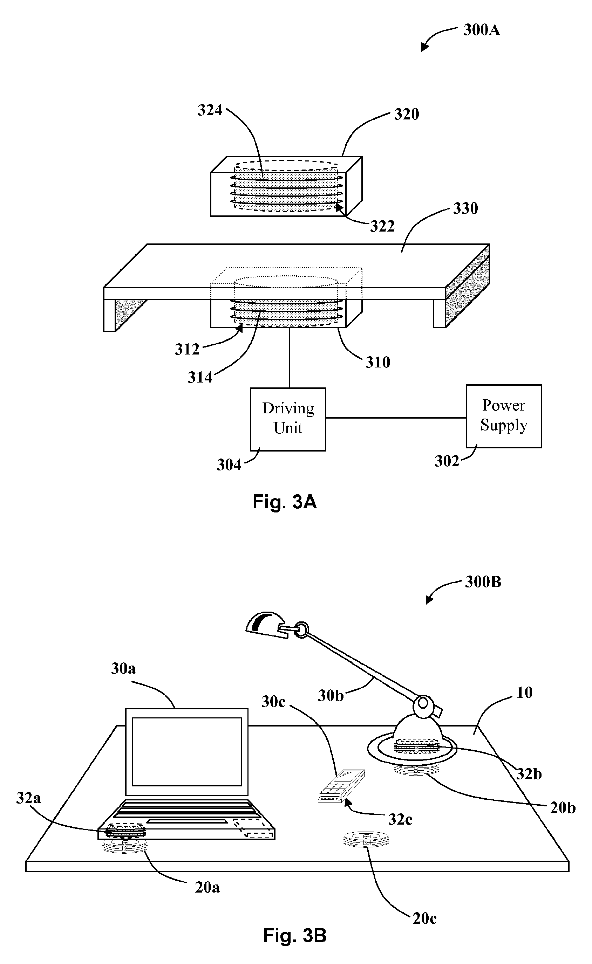

Reference is now made to Fig. 3A, there is provided a pin-less power coupling, which is generally indicated at 300A, according to one embodiment of the invention. A pin-less power jack 310, which may be incorporated into a substantially flat surface 330 for example, is couplable with a pin-less power plug 320. The pin-less power jack 310 includes an annular primary coil 312 shielded behind an insulating layer, which may be hardwired to a power source 302 via a driving unit 304. Driving electronics may include a switching unit providing a high frequency oscillating voltage supply, for example.

The pin-less power plug 320 includes an annular secondary coil 322 that is configured to inductively couple with the primary coil 312 of the pin-less power jack 310 to form a power transferring couple that is essentially a transformer. Optionally, a primary ferromagnetic core 314 is provided in the pin-less power jack 310 and a secondary ferromagnetic core 324 is provided in the pin-less power plug 320 to improve energy transfer efficiency.

It will be appreciated that known pinned power couplings of the prior art cannot be readily incorporated into flat surfaces. The nature of any pinned coupling is that it requires a socket into which a pin may be inserted so as to ensure power coupling. In contradistinction, the pin-less power coupling 300A of the second embodiment of the invention has no pin or socket and may, therefore, be incorporated behind the outer face of a flat surface 330, such as a wall, floor, ceiling, desktop, workbench, kitchen work surface, shelf, door or the like, at a location where it may be convenient to provide power.

It is specifically noted that because the primary coil 312 of the second embodiment is annular in configuration, alignment of the primary coil 312 to the secondary coil 322 is independent of the angular orientation of the pin-less power plug 320. This allows the pin-less power plug 320 to be coupled to the pin-less power jack 310 at any convenient angle to suit the needs of the user and indeed to be rotated whilst in use.

For example, a visual display unit (VDU) may draw its power via a pin-less power plug 320 of the second embodiment aligned to a pin-less power jack 310 of the second embodiment incorporated into a work desk. Because of the annular configuration of the coils 312, 322, the angle of the VDU may be adjusted without the pin-less coupling 300A being broken.

Prior art inductive coupling systems are not easily rotatable. For example, in order to achieve partial rotation, the system described in United States Patent No. 6,803,744, to Sabo, requires the coils to be connected by flexible wires or brushes to concentric commutators on the body of a non-conductive annular container. Even so, Sabo's system allows rotation of only about half the intercoil angle. In contradistinction, the pin-less power plug 120 of the second embodiment of the present invention may be rotated through 360 degrees or more, about the central axis of the annular primary coil 110 whilst continually maintaining the power coupling 300A.

It is known that inductive energy transfer is improved considerably by the introduction of a ferromagnetic core 314, 324. By optimization of the coupling 300A, appropriate electrical loads, such as standard lamps, computers, kitchen appliances and the like may draw power in the range of 10W - 200W for example.

Reference is now made to Fig. 3B, there is provided a schematic representation of an illustrative example of an inductively enabled platform, which is generally indicated at 300B, operable as a wireless transfer unit. The platform 10, which may be a table top, inductive mat or the like, includes a plurality of embedded inductive power transmitters' 20a-c. The inductive power transmitters' 20a-c are configured to transfer power inductively to inductive power receivers' 32a-c incorporated into various electrical appliances. A computer 30a is positioned such that an integrated inductive power receiver 32a is aligned to a first inductive power transmitter 20a, accordingly the first inductive power transmitter 20a may operate in tightly coupled mode. A desk lamp 30b is positioned such that its integrated inductive power receiver 32b is in alignment with a second inductive power transmitter 20b, accordingly the second power transmitter may also operate in tightly coupled mode. Thus power may be transferred to the computer 30a and the desk lamp 30b in an efficient manner with very little electromagnetic radiation leaking therefrom.

It will be noted that although a third inductive power transmitter 20c is available and a telephone 30c having an integrated inductive power receiver 32c is placed upon the platform, the telephone's inductive power receiver 32c is not aligned to the third inductive power transmitter 20c. It is a feature of the inductive power transfer system described herein that the inductive power transmitter 20c is capable of loosely coupling with a non-aligned inductive power receiver 32c such that the telephone 30c may be charged remotely.

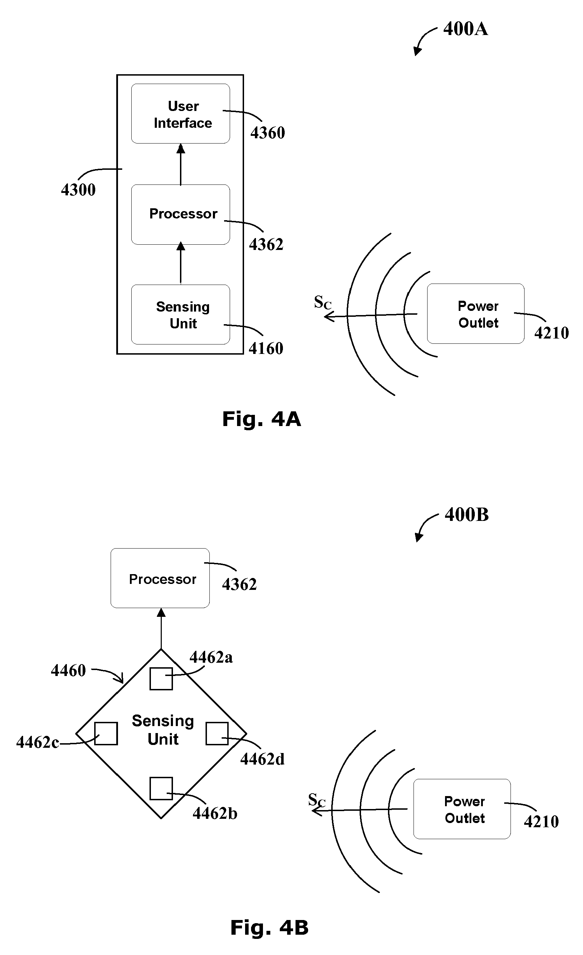

Reference is now made to Fig. 4A, there is provided a block diagram representing the main functional components of a wireless power locating mechanism, which is generally indicated at 400A.

The wireless power locating mechanism includes the wireless power outlet itself 4210 and an associated power outlet locator 4300. The power outlet locator 4300 comprising a sensing unit 4160 configured and operable to detect the wireless power outlet 4210 is provided. A processor 4362, in communication with the sensing unit 4160, is configured to compute the location of the power outlet 4210. A user interface 4360 is provided for communicating the computed location to a user.

According to various embodiments, the sensor unit 4160 may incorporate magnetic sensors such as Hall probes, for example, configured to detect the magnetic field generated by the wireless power outlet directly. Alternatively, the sensor unit 4160 may incorporate a radio receiver for receiving a radio signal transmitted from the wireless power outlet. It will be appreciated, however, that appropriate sensors may be selected for detecting specific electromagnetic wavelengths, including ultra-violet radiation, micro waves, radio waves or even x-ray or shorter wavelengths. Furthermore, the sensing unit may be configured to receive other types of radiation, including mechanical vibrations such as both audible and inaudible (e.g. ultrasonic) sound waves.

Reference is now made to Fig. 4B, there is provided a block diagram representing the functional components of a sensing unit, which is generally indicated at 400B.

It is noted that the block diagram 400B is showing by way of example, an exemplary sensing unit 4460 using four sensors 4462a-d, such as proximity sensors based on volume sensors, infra-red sensors, ultrasonic sensors, magnetic sensors (like Hall probes), inductance sensors, capacitance sensors or the like, are arranged in a diamond configuration.

Each sensor 4462 is configured to receive a control signal S_{C} transmitted from a wireless power outlet 4210. The processor 4362 may compare the intensity I of the control signal S_{C} detected by a sensor 4462 with a reference value Iᵣ to indicate the distance between the sensor 4462 and the wireless power outlet 4210.

Furthermore, the diamond configuration, provides two perpendicular opposing pairs of sensors 4462a-b, 4462c-d. The intensity I of the control signal S_{C} is measured by each sensor independently. The processor 4460 may use the differences between intensities measured by opposing pairs (Iₐ - I_{b}), (I_{c} - I_{d}) to provide vector coordinates indicating the direction of the power outlet 9210. Although a two dimensional vector is computed using the two dimensional diamond configuration of sensors described hereinabove, it will be appreciated that a three dimensional vector may be computed from three pairs of sensors in a tetrahedral configuration.

It will be appreciated that the computation method herein described are by way of example, for illustrative purposes only. Alternative methods by which the processor may compute the direction of the power outlet will be familiar to those skilled in the art.

It is noted that a digital bit-rate modulated control signal S_{C} may be used. Alternatively, the control signal S_{C} may alternatively be modulated in other ways such as by analogue or digital frequency modulation or by amplitude modulation, for example.

Reference is now made to Fig. 5, there is provided a partial schematic diagram of a power transfer system via a wireless power receiver, which is generally indicated at 500A, operable to monitor a sensor arrangement and perform associated efficiency analysis.

It is noted that it is a particular feature of certain embodiments of the invention that an inductive communications channel is incorporated into the inductive power transfer system, for transferring signals between a wireless power outlet and a wireless power receiver associated with an electrical hosting device. The communication channel is configured to produce an output signal in the wireless power outlet when an input signal Sᵢₙ is provided by the wireless power receiver 2300 without interupting the inductive power transfer from the power outlet to the wireless power receiver 2300.

In general, the wireless power outlet includes a primary inductive coil 2220 wired to a power source via a driver. The driver is configured to provide an oscillating driving voltage to the primary inductive coil, typically at a voltage transmission frequency fₜ which is higher than the resonant frequency f_{R} of the system.

The wireless power receiver 2300A includes a secondary inductive coil 2320 associated with a sensor arrangement 2128 configured to sense various power transfer parameters, a monitoring unit 2134 operable to monitor power transfer data from the sensor arrangement 2128 and a processor 2132. The secondary coil is wired to an electric load 2340, which is inductively coupled to the primary inductive coil 2220 of the wireless power outlet (partially shown). The electric load 2340 draws power from the power source 2240. Where the electric load 2340 requires a direct current supply, for example a charging device for an electrochemical cell or the like, a rectifier (not shown) may be provided to rectify the alternating current signal induced in the secondary coil 2320.

It is noted that the monitoring unit 2134 is operable to receive orientation data from the sensor arrangement 2128. Further, the monitoring unit 2134 is configured to analyze the power transfer parameters of each of the sensors of the sensor arrangement 2128 and further calculate efficiency data from the orientation data received from the sensor arrangement 2128.

An inductive communication channel may be provided for transferring signals from the secondary inductive coil 2320 using a communication and control unit (transmission circuit 2122) associated with the power receiver, to the primary inductive coil 2220 concurrently with uninterrupted inductive power transfer from the primary inductive coil 2220 to the secondary inductive coil 2320. The communication channel may provide feedback signals to the driver (not shown) of the power outlet.

The inductive communication channel may include a transmission circuit 2122 on the receiver side and a receiving circuit (not shown) on the transmitter side. The transmission circuit 2122 is wired to the secondary coil 2320, optionally via a rectifier (not shown), and the receiving circuit may be wired to the primary coil 2220.

The signal transmission circuit 2122 includes at least one electrical element 2126, selected such that when it is connected to the secondary coil 2320, the resonant frequency f_{R} of the system increases. The transmission circuit 2122 is configured to selectively connect the electrical element 2126 to the secondary coil 2320. As noted above, any decrease in either the inductance L or the capacitance C increases the resonant frequency of the system. Optionally, the electrical element 2126 may be have a low resistance for example, with a resistance say under 50 ohms and Optionally about 1 ohm.

It is further noted that in some embodiments the monitoring unit 2124 is physically incorporated within the wireless power receiver 2300. The monitoring unit 2124 communicates with the processor 2132 via a signal transfer system comprising a signal transmitter incorporated within the electrical device which is configured to transmit a signal to a signal receiver incorporated within the power receiver 2300.

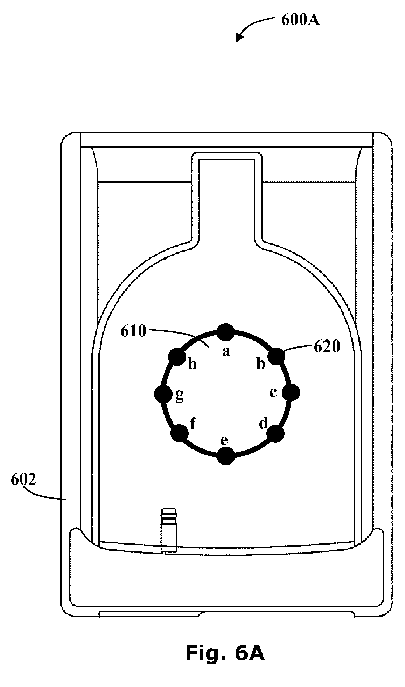

Reference is now made to Fig. 6A, there is provided elements of a wireless power receiver, which is generally indicated at 600A, including a secondary coil of and an associated sensor arrangement.

The wireless power receiver 600A is incorporated into an electrical hosting device 602 and the secondary coil 610 associated with the power receiver comprising a sensor arrangement 620. By way of example only, the exemplified sensor arrangement a-h is equally spaced in a circle. It is specifically noted that the other sensor arrangement may be applicable, depending the shape and type of the power receiver and associated secondary coil.

It is noted that using various sensor arrangement associated with the secondary coil of the power receiver, the orientation (location, direction and displacement) secondary coil relative to the primary coil of the power outlet may be determined. More specifically, by monitoring the sensors' power transfer parameters such as voltage, current, capacity, resistance, temperature and the like and by knowing the location of each sensor, the power receiver may calculate to determine the associated orientation of the power receiver with respect to the power transmitter.

It is further noted that various sensors may be used, such as a sensing coil, a magnetic field sensor, a temperature sensor, a position sensor and the like.

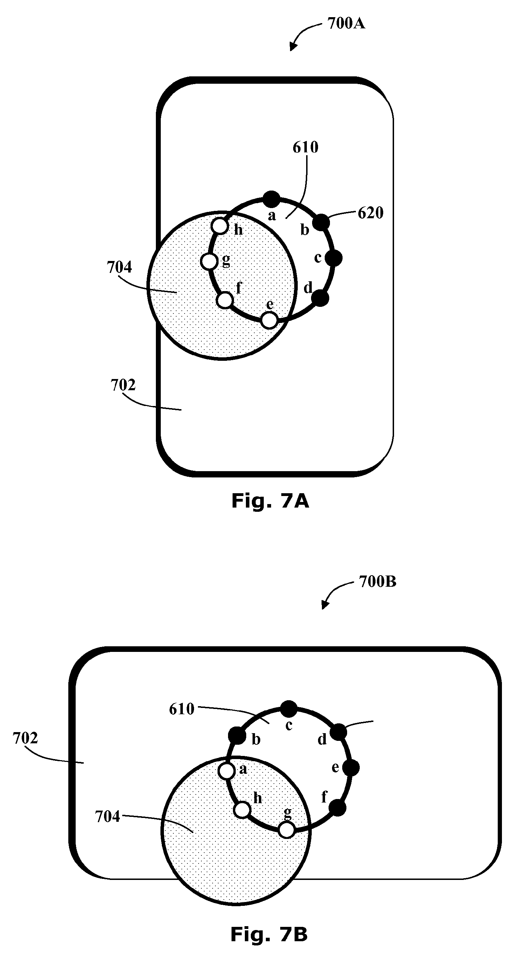

Reference is now made to Figs. 7A-B, there is provided two different orientations of a secondary coil associated with a wireless power receiver relative to a primary coil associated with a wireless power outlet, which is generally indicated at 700A and 700B, including the associated sensor arrangement.

It is noted that the orientation as specified in Figs. 7A differ only in terms of the direction. The location of the secondary coil relative to the primary coil is the same in both figures. Similarly, the displacement between the coils is the same in Fig. 7A and Fig. 7B.

The wireless power receiver is incorporated into an electrical hosting device 702 and the secondary coil 710 associated with the power receiver comprising a sensor arrangement 720 represented with sensor array a-h, equally spaced in a circle by way of example only.

The first orientation 700A, in Fig. 7A, represents an first orientation having a first location, a first direction (vertical) and first displacement (may be measure in terms of vertical and horizontal offsets) of the secondary coil relative to the primary coil. In the first orientation the sensors a-d are positioned outside a common area of the two coils and sensors e-h are within the common area. Similarly, the second orientation 700B, in Fig. 7B, having a second location, a second direction (horizontal) and a second displacement, with the the sensors b-f positioned outside the common area of the two coils and sensors a, h and g are within the common area.

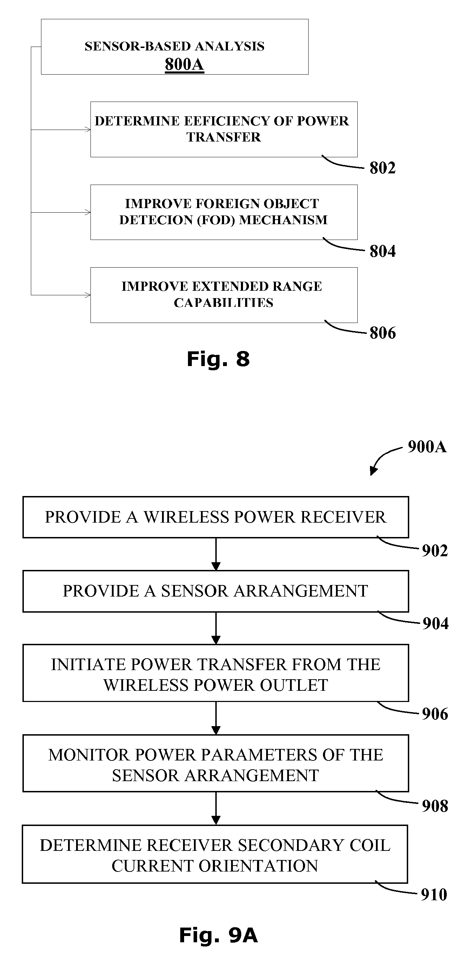

Reference is now made to Fig. 8, there is provided a diagram representing possible various efficiency related sensor-based analysis, which is generally indicated at 800A, for determining/improving efficiency of wireless power transfer on the wireless power receiver side.

It is noted, in particular, that existing methods rely on determining the displacement of coils (primary / secondary) for a single coil architecture, or determining location for multi-coil array architecture performed by the wireless power outlet (Tx) side, only. The current disclosure provides for a novel method to enable the wireless power receiver (Rx) associated with an electrical hosting device such as a mobile phone, a tablet and the like, to determine its own location relative to the wireless power transmitter's primary coil and also the associated orientation and, further to estimate the electrical power losses and indicate the desired location / orientation to enable efficient wireless power charging.

For example, the metallic components both in the Rx receiver and in the electrical hosting device may decrease the efficiency of power transfer, resulting in slow charging process. Furthermore, as the metallic component's arrangement in an electrical hosting device is not always symmetric, different orientations of the electrical hosting device may result with different levels of electrical power losses. Thus, the current disclosure provides the tools to determine the orientation and location of the electrical hosting device (specifically of the secondary coil) and further compute accurate estimation for the expected electrical poser losses for every location and every orientation of the electrical hosting device.

The diagram 800A for efficiency related sensor-based analysis, may include activity step performed by the monitoring unit associated with the wireless power receiver, using an associated processing unit. The diagram includes the steps:
In step 802 - determining the efficiency of wireless power transfer associated with the wireless power receiver side;
In step 804 - improving the foreign object detection (FOD) mechanism, may increase the wireless power transfer efficiency. As described hereinabove, the metallic components of the power receiver and the electrical hosting device may interfere with the power transfer. Thus, a design of the hosting device in such a way that the metallic components may be placed as far as possible from the power receiver's coil may improve the efficiency of wireless power transfer a better. Another possible solution may use a metallic shielding between the Rx power receiver and the hosting device in order to minimize influence of the metallic components; and
In step 806 - improving extended range capabilities.

It is noted that the processing unit may be the processor of the electrical hosting device. Additionally or alternatively, the processing unit may be a separate dedicated unit and part of the wireless power receiver.

Reference is now made to Fig. 9A, there is provided a flowchart representing a possible method, which is generally indicated at 900A, for use in a wireless power receiver wherein the secondary coil is associated with a sensor arrangement and further configured to determine secondary coil orientation.

It is noted that the wireless power receiver includes a secondary coil connectable to an electric load and operable to couple with a primary coil associated with a wireless power outlet; a communication and control unit operable to communicate power control signals to said wireless power outlet; a plurality of electrical sensors associated with the secondary coil configured to sense an orientation of the secondary coil relative to the primary coil; and a monitoring unit operable to communicate with a processing unit, the monitoring unit configured to monitor power transfer parameters associated with each of the plurality of electrical sensors.

The method 900A is for operating a wireless power receiver used within an associated hosting electrical device. The secondary coil of the wireless power receiver is operable to couple with a primary coil associated with a wireless power transmitter and configured to transmit communication signals to trigger wireless power transfer. The method 800B includes the following steps:
In step 902 - providing a wireless power receiver associated with an electric hosting device;
In step 904 - providing a sensor arrangement associated with the secondary coil of the wireless power receiver;
In step 906 - initiating power transfer from the wireless power outlet to the electrical hosting device via the wireless power receiver. The power transfer may be triggered by the communication and control unit operable to communicate power control signals to the wireless power outlet; and
In step 908 - monitoring the power transfer parameters for each sensor of the sensor arrangement; and
In step 910 - determining the current orientation of the secondary coil associated with the wireless power receiver relative to the primary coil associated with the wireless power outlet.

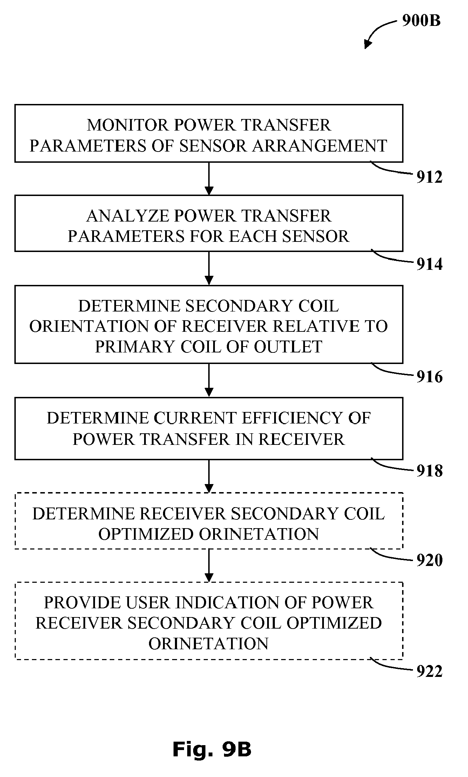

Reference is now made to Fig. 9B, there is provided a flowchart representing a possible method, which is generally indicated at 900B, for use in a wireless power receiver analyzing input power data received from a sensor arrangement and further configured to perform efficiency data analysis.

The method 900B is for operating a wireless power receiver used within an associated hosting electrical device efficiently. The secondary coil of the wireless power receiver is operable to couple with a primary coil associated with a wireless power transmitter and configured to analyze efficiency data retrieved from a sensor arrangement associated with the secondary coil. The method 900B includes the following steps:
In step 912 - monitoring the power transfer parameters for each sensor of the sensor arrangement, associated with the secondary coil of the wireless power receiver;
In step 914 - analyzing the power transfer data of each sensor of the sensor arrangement to enable efficiency calculation regarding the associated electrical power loss;
In step 916 - determining the orientation of the secondary coil associated with the power receiver relative to the primary coil associated with the coupled wireless power outlet;
In step 918 - determining the current efficiency of wireless power transfer associated with the wireless power receiver in terms of the level of electrical power loss;
In step 920 - optionally, determining the optimized orientation of the secondary coil associated with the wireless power receiver relative to the primary coil associated with the wireless power outlet; and
In step 922 - optionally, providing user indication of the optimized orientation of the secondary coil relative to the primary coil. Additionally or alternatively, providing user indication of the current orientation of the secondary coil, for comparison purposes.

Optionally, the level of wireless power loss may also be indicated to the user. As appropriate, the level of electrical power loss and orientation may be indicated to the user via a user interface, visually such as a LED indicator, for example, or an audio interface such as a speaker or other such sound generator.

It is noted that determining the optimized orientation may requires repeated calculations of efficiency data of wireless power transfer per a sequence of orientations, thereby selecting the orientation with the lowest wireless power loss, indicating higher efficiency value. Alternatively, the optimized orientation may be determined such that the associated computed power transfer efficiency is beneath a preconfigured threshold value of wireless power loss.

### Tx-Rx Communication

Each electrical device may have a unique identifier, which may be referred to as a receiver identification (RxID), in the system that allows the recognition thereof. The RxID may be a MAC address. The management server may store user or mobile electrical device related information in addition to the RxID, such as power transfer related data, billing information, user credits or the like.

Where appropriate, wireless power outlets may have a unique identifier, which may be referred to as a transmitter identification (TxID), in the system that allows the recognition thereof.

For illustrative purposes only, possible methods for providing access to power for electrical devices in public spaces are presented hereinafter. The method may allow a user to transfer power or charge an electrical device such as a mobile phone, a tablet or the like from a portable wireless power transfer unit and may further allow a power provider to manage the power transfer, while gathering power transfer related information.

A user may place or connect an electrical device to a portable wireless power transfer unit. For example an inductively enabled device may be placed upon a portable wireless power transfer unit. Alternatively, or additionally, a power supply may be conductively connected to an electrical device.

The power access point may detect the electrical device connection. For example, wired connection may be detected by detecting the load and wireless connection may be detected using various remote sensors such as hall sensors, analog ping schemes or the like.

### Audio Communication

In one particular embodiment, the close communication channel between the device and power access point may be based upon audio signals sensed via a microphone of the electrical device, for example using specific ultrasonic or audible bands, between 20 hertz and 20 kilohertz, between 300 hertz and 20 kilohertz, above 20 kilohertz, between 20 kilohertz and 25 kilohertz, above 25 kilohertz say or the like. The audio signal may be emitted from a transceiver or an audio emitter such as a speaker or the like associated with the portable wireless power transfer unit. Many electrical devices, such as mobile phones and the like have microphone and software applications may have access to the microphone.

It is noted that powering the microphone unit may itself demand power. Consequently, the software application running on the electrical device may activate the microphone only where 'a-charge-connect' event is detected in the system. Accordingly, upon detection of the device, the wireless power outlet may provide an initial power transfer to power the microphone. After a short interval, an identification signal may be sent via the audio signal.

The audio signal may include additional tones that are not related to the communication pattern which may mask the random patterns communicated. For example, an audio identification signal may be masked by a connection tone serving to provide users with an indication that a connection has been made.

### Data-over-coil (DOC) Communication

Alternatively or additionally, the close communication channel may be provided by the wireless power outlet alternating the activation of power transfer to the electrical device. The alternation of power supply is detected by most electrical devices as power transfer connection and disconnection events that are communicated to the application layer on these electrical devices.

The switching pattern may be coded with an identification signal such as the random pattern. The wireless power outlet may need to perform this switching in intervals spaced sufficiently apart to allow the electrical devices to detect and report to application level power transfer connection and disconnection events.

### Bluetooth and NFC

Still other embodiments may use Bluetooth, Low Energy Bluetooth or Near Field Communication (NFC) to achieve the close communication channel. These could be combined with the basic power signal to trigger their activation thereby conserving power.

Accordingly the wireless power transmission and receiving units may include a BLE radio transmitter operable to transmit between -6 and 8.5 dBm and a BLE receiver having a sensitivity of say -77dBm or better, for example as measured at the antenna connector.

In various embodiments of this system the LAN/WAN interface of the device may be WLAN or Cellular 2G/3G/4G connections. The connection to the WLAN or Cellular access point may also include manual or automatic insertion of user credentials. In this case the information may be conveyed to the management server to enable user identification. The information provided in order to allow access may also be stored by the device application and later provided directly to the management server.

Additionally, or alternately the LAN/WAN connection of the wireless power outlet may be achieved via the charged device. The wireless power outlet may encrypt messages to the management server and deliver this to the application on the electrical device via the close communication channel therebetween. The application may then send the message to the server via its LAN/WAN connection.

It is noted that there are various possible ways to communicate between the power receiver (Rx) and the power transmitter (Tx), including Infra-Red (IR) and any other known communication channels. Optionally, the choice of the actual communication channel may be implementation dependent.

Technical and scientific terms used herein should have the same meaning as commonly understood by one of ordinary skill in the art to which the disclosure pertains. Nevertheless, it is expected that during the life of a patent maturing from this application many relevant systems and methods will be developed. Accordingly, the scope of the terms such as computing unit, network, display, memory, server and the like are intended to include all such new technologies a priori.

As used herein the term "about" refers to at least ± 10 %.

The terms "comprises", "comprising", "includes", "including", "having" and their conjugates mean "including but not limited to" and indicate that the components listed are included, but not generally to the exclusion of other components. Such terms encompass the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" may include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments or to exclude the incorporation of features from other embodiments.

The word "optionally" is used herein to mean "is provided in some embodiments and not provided in other embodiments". Any particular embodiment of the disclosure may include a plurality of "optional" features unless such features conflict.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween. It should be understood, therefore, that the description in range format is merely for convenience and brevity and should not be construed as an inflexible limitation on the scope of the disclosure. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6 as well as non-integral intermediate values. This applies regardless of the breadth of the range.

It is appreciated that certain features of the invention, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. Certain features described in the context of various embodiments are not to be considered essential features of those embodiments, unless the embodiment is inoperative without those elements.

Although the invention has been described in conjunction with specific embodiments thereof, it is evident that other alternatives, modifications, variations and equivalents will be apparent to those skilled in the art. Accordingly, it is intended to embrace all such alternatives, modifications, variations and equivalents that fall within the spirit of the invention and the broad scope of the appended claims.

Additionally, the various embodiments set forth hereinabove are described in terms of exemplary block diagrams, flow charts and other illustrations. As will be apparent to those of ordinary skill in the art, the illustrated embodiments and their various alternatives may be implemented without confinement to the illustrated examples. For example, a block diagram and the accompanying description should not be construed as mandating a particular architecture, layout or configuration.

The presence of broadening words and phrases such as "one or more," "at least," "but not limited to" or other like phrases in some instances shall not be read to mean that the narrower case is intended or required in instances where such broadening phrases may be absent. The use of the term "module" does not imply that the components or functionality described or claimed as part of the module are all configured in a common package. Indeed, any or all of the various components of a module, whether control logic or other components, can be combined in a single package or separately maintained and can further be distributed in multiple groupings or packages or across multiple locations.

Furthermore, embodiments may be implemented by hardware, software, firmware, middleware, microcode, hardware description languages, or any combination thereof. When implemented in software, firmware, middleware or microcode, the program code or code segments to perform the necessary tasks may be stored in a computer-readable medium such as a storage medium. Processors may perform the necessary tasks.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present disclosure. To the extent that section headings are used, they should not be construed as necessarily limiting.

The scope of the disclosed subject matter is defined by the appended claims and includes both combinations and sub combinations of the various features described hereinabove as well as variations and modifications thereof, which would occur to persons skilled in the art upon reading the foregoing description.

## Claims

1. Wireless power receiver connectable with an electrical hosting device and configured to draw power from a wireless power outlet (200), said wireless power receiver comprising:
a secondary coil connectable (320; 2320) to an electric load (340) operable to couple with a primary coil (220) associated with said wireless power outlet (200);
a communication and control unit (2122) operable to communicate power control signals to said wireless power outlet (200);
a plurality of electrical sensors (4462) associated with said secondary coil (320; 2320) and configured to sense an orientation of the secondary coil (320) relative to the primary coil (220); and
a monitoring unit (2134) operable to communicate with a processing unit (2132), said monitoring unit configured to monitor power transfer parameters associated with each of said plurality of electrical sensors;
wherein said monitoring unit (2134) is operable to receive orientation data from said plurality of electrical sensors (4462), to calculate efficiency data from said sensor data and further operable to communicate said efficiency data to said communication and control unit.

2. Wireless power receiver of claim 1, wherein said plurality of electrical sensors (4462) are selected from a group consisting of: a sensing coil, a magnetic field sensor, a temperature sensor, a position sensor and combinations thereof.

3. Wireless power receiver of claim 1 or 2, wherein said orientation data comprises data pertaining to at least one of: at least one parameter determining a location, at least one parameter determining a direction and at least one parameter determining a displacement.

4. Wireless power receiver of one of claims 1 to 3, wherein said efficiency data determines an estimated level of electrical power loss at said orientation.

5. Wireless power receiver of one of claims 1 to 4, which is further operable to determine a desired orientation such that said estimated electrical power loss is less than a pre-configured efficiency threshold.

6. Wireless power receiver of one of claims 1 to 5, wherein said communication and control unit (2122) is further configured to provide a user indication of said orientation.

7. Wireless power receiver of one of claims 1 to 6, which is further operable to determine a desired orientation such that said estimated electrical power loss is beneath a pre-configured efficiency threshold.

8. Wireless power receiver of one of claims 1 to 7, wherein said power transfer parameters are selected from a group consisting of: a voltage parameter, a current parameter, a capacitance parameter, a resistance parameter, a temperature value and combinations thereof.

9. Wireless power receiver of one of claims 1 to 8, wherein said processing unit (2132) is associated with said electrical hosting device.

10. Wireless power receiver of one of claims 1 to 9, wherein said processing unit (2132) is associated with said wireless power receiver.

11. Wireless power receiver of one of claims 1 to 10, further comprising a metal shielding between said wireless power receiver and said electrical hosting device, said metal shielding operable to reduce the electric power loss associated with metallic components of said electrical hosting device beneath an efficiency threshold setting.

12. Method for use in a wireless power receiver (32a, 32b, 32c) associated with a hosting electrical device, said wireless power receiver operable to couple with a wireless power transmitter (20a, 20b, 20c) and configured to transmit communication signals to trigger wireless power transfer, said wireless power receiver (32a, 32b, 32c) comprises:
a secondary coil (320; 2320) connectable to an electric load operable to couple with a primary coil (220) associated with said wireless power outlet;
a communication and control unit (2122) operable to communicate power control signals to said wireless power outlet;
a plurality of electrical sensors (4462) associated with said secondary coil configured to sense an orientation of the secondary coil relative to the primary coil; and
a monitoring unit operable (2134) to communicate with a processing unit (2132), said monitoring unit configured to monitor power transfer parameters associated with each of said plurality of electrical sensors;
said method for operating said wireless power receiver in a manner such that the electrical power loss associated with said wireless power receiver (32a, 32b, 32c) during wireless power transfer are below a configurable threshold, the method comprising the steps of:
said communication and control unit (2122), coupling with said wireless power transmitter, triggering wireless power transfer from said wireless power transmitter (20a, 20b, 20c);
said monitoring unit (2134), receiving orientation data from said plurality of electrical sensors (4462), analyzing said power transfer parameters of each of said plurality of electrical sensors (4462) and calculating efficiency data from said orientation data received.

13. Method of claim 12, wherein said orientation data comprises data pertaining to at least one of: a location of said secondary coil relative to primary coil; a direction of said secondary coil relative to primary coil; and a displacement of said secondary coil relative to primary coil.

14. Method of claim 12 or 13, further comprising:
providing an indication of an estimated level of electrical power loss associated with said wireless power receiver, from said calculated efficiency data at the time of wireless power transfer, using a user interface; and/or
providing an indication of a current orientation of said secondary coil associated with said wireless power receiver relative to said primary coil associated with said wireless power transmitter, using a user interface; and/or
calculating an optimized orientation of said secondary coil relative to said primary coil, said optimized orientation having an estimated level of electrical power loss beneath a beneath an efficiency threshold setting and optionally providing an indication of said optimized orientation, using a user interface and/or an indication of said estimated level of electrical power loss and said efficiency threshold setting, using a user interface.

15. Method of any of claims 12 to 14,
further using at least one of a visual interface or an audio interface as a user interface; and/or
selecting said power transfer parameters from a group consisting of: a voltage parameter, a current parameter, a capacitance parameter, a resistance parameter, a temperature parameter and combinations thereof.
